(19) Europäisches Patentamt
European Patent Office
Office européen des brevets

(11) **EP 3 632 306 A1**

(12) **EUROPEAN PATENT APPLICATION**
published in accordance with Art. 153(4) EPC

(43) Date of publication:
**08.04.2020 Bulletin 2020/15**

(51) Int Cl.:
**A61B 5/055** (2006.01)   **A61B 5/00** (2006.01)

(21) Application number: **18808734.0**

(22) Date of filing: **10.05.2018**

(86) International application number:
**PCT/JP2018/018115**

(87) International publication number:
**WO 2018/221152 (06.12.2018 Gazette 2018/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.05.2017 JP 2017106643**

(71) Applicant: **Kyushu University, National University Corporation**
**Fukuoka-shi, Fukuoka 819-0395 (JP)**

(72) Inventors:
• **ARIMURA Hidetaka**
  **Fukuoka-shi, Fukuoka 819-0395 (JP)**
• **JIN Ze**
  **Fukuoka-shi, Fukuoka 819-0395 (JP)**
• **HIRAKAWA Kazuya**
  **Fukuoka-shi, Fukuoka 819-0395 (JP)**

(74) Representative: **EP&C**
**P.O. Box 3241**
**2280 GE Rijswijk (NL)**

(54) **METHOD FOR GENERATING MAP INFORMATION, DETERMINATION METHOD, AND PROGRAM**

(57)    A method for generating map information includes: a thinned patient image generating step of generating thinned patient image information in which coordinate information of brain blood vessels of a patient having a cerebral aneurysm and coordinate information of cerebral aneurysms are associated with each other, and the brain blood vessels are thinned; a coordinate associating step of associating coordinates of thinned blood vessels represented by the thinned patient image with reference coordinates of brain blood vessels represented by a thinned reference image in which the brain blood vessels are thinned; and a map information generating step of generating probabilistic map information representing probabilities of occurrence in the coordinates of cerebral aneurysms with respect to the reference coordinates on the basis of the coordinates of the brain blood vessels associated in the coordinate associating step for the thinned patient image information of a plurality of patients having cerebral aneurysms and the coordinate information.

*FIG. 2*

EP 3 632 306 A1

**Description**

[Technical Field]

The present invention relates to a method for generating map information, a determination method, and a program.

Priority is claimed on Japanese Patent Application No. 2017-106643, filed May 30, 2017, the content of which is incorporated herein by reference.

[Background Art]

The prevalence rate of intracranial aneurysms (hereinafter, referred to as cerebral aneurysms) has been estimated as being in the range of 3.6 percent to 6.0 percent based on anatomical predictions and examinations according to angiography. A cerebral aneurysm is a cerebrovascular disorder in the brain and causes local expansion of blood vessels or ballooning of vessels. Rupturing of a cerebral aneurysm causes subarachnoid hemorrhage and intracranial bleeding. The mortality rate from subarachnoid hemorrhage or intracranial bleeding is in the range of 40 percent to 50 percent.

In recent years, great interest in the prevention of cerebral strokes and dementia has led to the establishment of medical examination systems for intracranial diseases and cerebrovascular diseases. Medical examinations known also as a brain checkup are widely performed in Japan. It was disclosed in a nationwide survey that by 2009, brain checkups could be performed in 235 accredited medical institutions in Japan. Cerebral aneurysms were detected in 2.6 percent of persons who have received examinations through brain checkups.

As a standard inspection method used for a cerebral aneurysm screening inspection, there is magnetic resonance angiography (MRA). MRA is used for forming an image of blood vessels in order to detect cerebral aneurysms, stenosis, obstructions, or other abnormalities of brain blood vessels. MRA has advantages of low toxicity and non-invasiveness due to a contrast agent not being used over angiography using computed tomography (CT) or catheter angiography and thus is widely used.

In maximum intensity projection (MIP) used for processing an MRA image, there are cases in which images overlap each other, or a cerebral aneurysm is present at an unusual position. For this reason, it may be difficult for a radiological doctor to manually detect a cerebral aneurysm.

In order to assist radiological doctors, many computer-aided diagnostic (CAD) system frameworks have been developed for detection of asymptomatic unruptured cerebral aneurysms using an MRA image. According to research, it has been shown statistically that when using CAD systems there is a significant improvement in enhancing diagnostic results of radiological doctors having relatively little experience and shortening of an image reading time.

An aneurysm determination method, a device thereof, and a computer program in which inflow-side blood vessels and outflow-side blood vessels of parent blood vessels of an aneurysm are designated and marked, blood vessel core lines are extracted through thinning processing, and abnormalities such as expansion and the like of blood vessels are identified are known (for example, see Patent Literature 1).

[Citation List]

[Patent Literature]

[Patent Literature 1]
Japanese Unexamined Patent Application, First Publication No. 2009-240543

[Summary of Invention]

[Technical Problem]

When identifying the location of cerebral aneurysms, a map representing the probability of occurrence of cerebral aneurysms may be used. The generation of such a map needs to be performed manually by an expert using a database, but there is a problem of the time required for this. In a method for identifying the location of cerebral aneurysms using a map representing the probability of occurrence of cerebral aneurysms, a technology for automatically generating a map representing the probability of occurrence of cerebral aneurysms is required.

The present invention is realized in view of the points described above and provides a method for generating map information, a determination method, and a program capable of automatically generating a map that represents a probability of occurrence of cerebral aneurysms.

[Solution to Problem]

(1) The present invention is capable of solving the problems described above, and an aspect of the present invention is a method for generating map information including: a thinned patient image generating step of generating thinned patient image information in which coordinate information of brain blood vessels of a patient having a cerebral aneurysm and coordinate information of cerebral aneurysms are associated with each other, and the brain blood vessels are thinned; a coordinate associating step of associating coordinates of thinned blood vessels represented by the thinned patient image information generated in the thinned patient image generating step with reference coordinates of brain blood vessels represented by a thinned reference image in which the brain blood vessels are thinned; and a map information generating step of generating probabilistic map information representing probabilities of occurrence in the coordinates of cerebral aneurysms with respect to the reference coordinates on the basis of the coordinates of the brain blood vessels associated in the coordinate associating step for the thinned patient image information of a plurality of patients having cerebral aneurysms and the coordinate information.

(2) In addition, according to an aspect of the present invention, in the method for generating map information described above, the probabilistic map information is generated on the basis of sizes of the cerebral aneurysms in the map information generating step.

(3) In addition, an aspect of the present invention is a determination method including: a determination target thinned patient image generating step of generating determination target thinned patient image information in which coordinate information of brain blood vessels of a patient having a cerebral aneurysm that is a determination target and coordinate information of cerebral aneurysms are associated with each other, and the brain blood vessels are thinned; a probabilistic map information generating step of generating the probabilistic map information using the method for generating map information; a second coordinate associating step of associating coordinates of thinned brain blood vessels represented by the probabilistic map information generated in the probabilistic map information generating step with reference coordinates of the brain blood vessels represented by the determination target thinned patient image information; a second map information generating step of generating second probabilistic map information on the basis of the coordinates of the brain blood vessels associated in the second coordinate associating step and the coordinate information; and a false positive determining step of determining a false positive included in the second probabilistic map information using Bayesian estimation.

(4) In addition, according to an aspect of the present invention, in the determination method described above, a ratio between eigenvalues of Hessian matrices of quadratic equations representing ellipsoids when cerebral aneurysms are regarded as ellipsoids is used in the false positive determining step.

(5) In addition, an aspect of the present invention is a program causing a computer to execute: a thinned patient image generating step of generating thinned patient image information in which coordinate information of brain blood vessels of a patient having a cerebral aneurysm and coordinate information of cerebral aneurysms are associated with each other, and the brain blood vessels are thinned; a coordinate associating step of associating coordinates of thinned blood vessels represented by the thinned patient image information generated in the thinned patient image generating step with reference coordinates of brain blood vessels represented by a thinned reference image in which the brain blood vessels are thinned; and a map information generating step of generating probabilistic map information representing probabilities of occurrence in the coordinates of cerebral aneurysms with respect to the reference coordinates on the basis of the coordinates of the brain blood vessels associated in the coordinate associating step for the thinned patient image information of a plurality of patients having cerebral aneurysms and the coordinate information.

[Advantageous Effects of Invention]

According to the present invention, a map that represents a probability of occurrence of cerebral aneurysm can be automatically generated.

[Brief Description of Drawings]

Fig. 1 is a diagram illustrating one example of a distribution of cerebral aneurysms and false positives in a brain blood vessel image according to an embodiment.
Fig. 2 is a diagram illustrating one example of the process of automatically generating a cerebral aneurysm probabilistic atlas according to this embodiment.

Fig. 3 is a diagram illustrating one example of a thinned image of major blood vessels as a reference and a thinned image of major blood vessels of a clinical case before alignment is performed according to this embodiment.

Fig. 4 is a diagram illustrating one example of a thinned image of major blood vessels as a reference and a thinned image of major blood vessels of a clinical case after positioning is performed according to this embodiment.

Fig. 5 is a diagram illustrating one example of a cerebral aneurysm probabilistic atlas according to this embodiment.

Fig. 6 is a diagram illustrating one example of a Bayesian CAD framework using a cerebral aneurysm probabilistic atlas according to this embodiment.

Fig. 7 is diagram illustrating one example of a distribution of ratios of eigenvalues for false positives and cerebral aneurysms according this embodiment.

Fig. 8 is a diagram illustrating one example of an image acquired by viewing a cerebral aneurysm probabilistic atlas from the front side to the rear side according to this embodiment.

Fig. 9 is a diagram illustrating one example of a FROC curve of a Bayesian CAD framework according to this embodiment.

Fig. 10 is a diagram illustrating one example of the functional configuration of a Bayesian CAD framework using a cerebral aneurysm probabilistic atlas according to this embodiment.

[Description of Embodiments]

(Embodiment)

Hereinafter, an embodiment of the present invention will be described in detail with reference to the drawings.

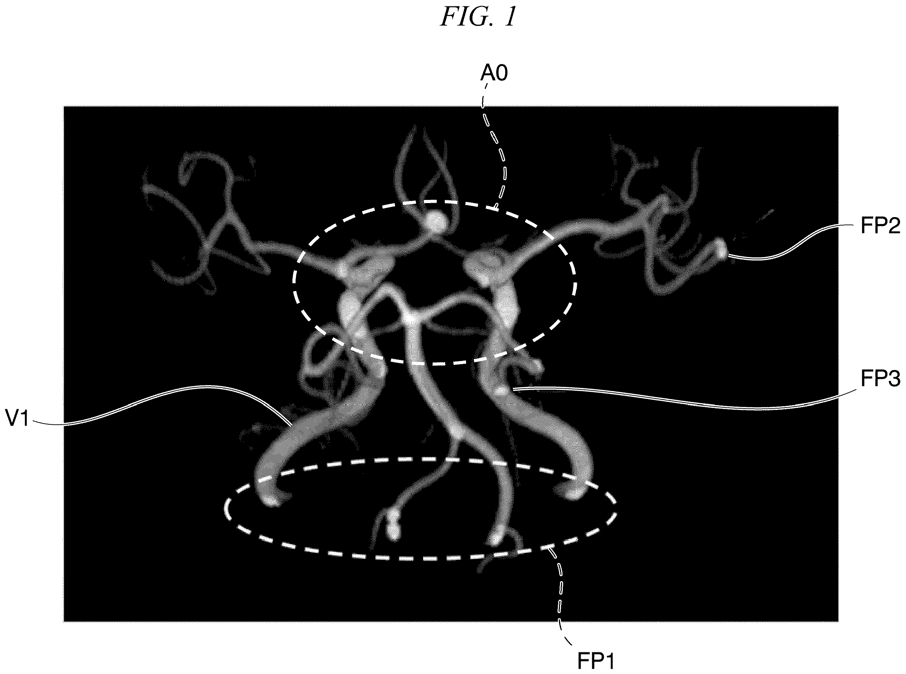

Fig. 1 is a diagram illustrating one example of a distribution of cerebral aneurysms and false positives in a brain blood vessel image according to an embodiment. The brain blood vessel image is an image of brain blood vessels in a case in which a brain is viewed from the side in front.

In a conventional diagnosis in a CAD system, various methods have been developed in order to detect a cerebral aneurysm. As one example, an ellipsoid convex enhancement (ECE) filter that can decrease false positives in bending parts of blood vessels and selectively enhance shapes of cerebral aneurysms has been proposed. By using the ECE filter, the number of false positives is decreased by up to 51.4 percent.

However, as illustrated in Fig. 1, several false positives may be detected in an entrance part FP1 of an image area in an imaged blood vessel V1 or terminal ends FP2 and FP3 of the imaged blood vessel VI. In addition, true positives may be detected in a location A0.

In order to further decrease the number of false positives, prior information of locations of cerebral aneurysms based on past diagnoses and a database that currently exists is useful for generation of a probabilistic atlas that is a map representing a probability of occurrence of the cerebral aneurysm. It is known that most cerebral aneurysms occur surrounding the cerebral artery circle.

A probabilistic atlas has been used for a small piece of an organ such as a liver and the structure of a brain. However, there are few known methods using prior information of common locations at which cerebral aneurysms occur.

In this embodiment, a method of automatically generating a probabilistic atlas of cerebral aneurysms from a database using a computer is provided. In a technique using a computer, prior information of locations of cerebral aneurysms is used.

The shapes of blood vessels differ from individual to individual while the locations of cerebral aneurysms depend on the structure of blood vessels and the structures of blood vessels have a common pattern. It is difficult to perform aligning of reference data with a plurality of pieces of clinical data using only rigid body registration. In this method, thin plate spline - robust point matching (TPS-RPM) that is a technique for non-rigid registration is used for alignment between major blood vessels of brain blood vessels.

In this embodiment, information of locations of cerebral aneurysms acquired in 89 non-ruptured cerebral aneurysms of 72 patients is used for generation of a probabilistic atlas of cerebral aneurysms. In addition, in this embodiment, a CAD framework decreasing the number of false positives on the basis of Bayesian estimation combining a probabilistic atlas of cerebral aneurysms and an ECE filter is handled.

The probabilistic atlas of cerebral aneurysms according to this embodiment will be referred to as a probabilistic atlas PA.

(Clinical cases)

A database according to this embodiment includes data of 72 patients, who have 89 non-ruptured cerebral aneurysms in total. Here, the 72 patients are persons including 17 males and 55 females whose ages are 36 to 89. The database includes data of one healthy person as reference data. The reference data is data from females aged 45 who received MRA examination in the years 2006 to 2007 or 2010 to 2014. The reference data was selected from data of 30 persons who were not suffering from cerebral aneurysms by using the reference of a shape of blood vessels of a healthy person.

The cerebral aneurysms included in the database were checked by two experienced neuroradiographic doctors using CT angiography or digital subtraction angiography (DSA) in accordance with the guidelines of The Japan Stroke Society. MRA images of patients were acquired using a 3.0 tesla magnetic resonance image scanner. A three-dimensional MRA image includes 112 to 172 slices per case. Here, each of the slices is a slice having a thickness of 1 millimeters to 1.2 millimeters and a length of 0.5 millimeters to 0.7 millimeters. An image of the slice is composed of 512 x 512 pixels having a pixel size of 0.3516 millimeters. The original three-dimensional MRA image is converted into isotropic volume data formed from a matrix having a size of $512 \times 512 \times (224 - 258)$ and isotropic voxels having a size of 0.3516 millimeters by using a three-dimensional interpolation method.

A long diameter and two short diameters of a cerebral aneurysm were manually measured respectively in accordance with a longest axis, an intermediate axis, and a shortest axis of each ellipsoid. Here, the longest axis, the intermediate axis, and the shortest axis of the ellipsoid are orthogonal to one another. The measurement was performed twice on an MRA image by each of two measurers using multi-planar reconstruction (MPR) software. The long diameter of 89 cerebral aneurysms took a value in the range of 1.4 millimeters to 10.6 millimeters, and an average value was 4.4 millimeters.

A distribution of locations of cerebral aneurysms according to this embodiment will be described.

40 percent of cerebral aneurysms occur in middle cerebral arteries (MCA), and 38 percent of cerebral aneurysms occur in internal carotid arteries (ICA) and ICA-posterior communicating arteries (ICA-Pcom). In this way, most cerebral aneurysms occur in MCAs, ICAs, and ICA-Pcoms.

10 percent of cerebral aneurysms occur in anterior communicating arteries (Acom). 5 percent of cerebral aneurysms occur in anterior cerebral arteries (ACA). 3 percent of cerebral aneurysms occur in the tips of basilar arteries (BAtip) or basilar artery-superior cerebellar arteries (BA-SCA) branching parts. 2 percent of cerebral aneurysms occur in vertebral arteries - posterior inferior cerebella arteries (VA-PICA) branching parts.

(Generation of cerebral aneurysm probabilistic atlas)

A probabilistic atlas PA is determined using a Gauss sphere representing locations and sizes of cerebral aneurysms of a database in a blood vessel image serving as a reference. A location of each cerebral aneurysm in the blood vessel image serving as a reference is determined by converting a location of the cerebral aneurysm in a clinical image into a location in the blood vessel image serving as the reference by using a deformation vector field. Here, the deformation vector field is generated by aligning thinned images of major blood vessels of a clinical case and the blood vessel image serving as the reference by using the TPS-RPM method. Thereafter, the Gauss sphere is disposed at the location of the cerebral aneurysms for which positioning has been performed. Here, a standard deviation of the Gaussian distribution of the Gauss sphere is 1/4 of the value of the long diameter of each cerebral aneurysm.

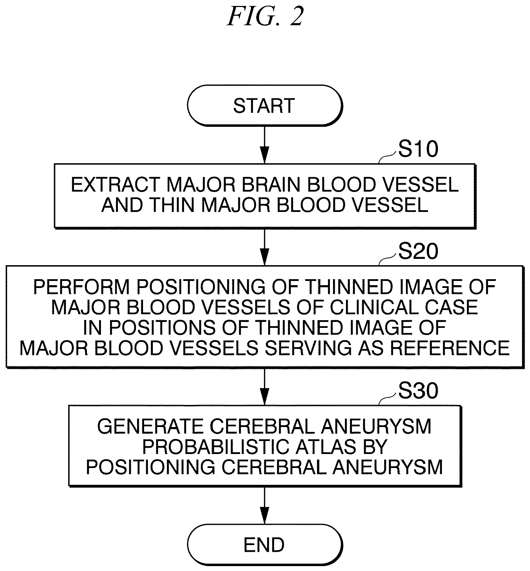

Fig. 2 is a flowchart illustrating the flow of a process of generating a probabilistic atlas PA from a database. In this embodiment, a method of generating a probabilistic atlas PA will be referred to as a map information generating method M.

(Step S 10) A thinned image of major brain blood vessels is generated from an MRA image.

In order to perform robust positioning of blood vessels, the thinned images of the major blood vessels are extracted. Here, a reason for using major blood vessels is that the major blood vessels are surrounded by locations at which cerebral aneurysms commonly occur, and a distribution of the major blood vessels has a lower uncertainty than that of a distribution of branched blood vessels.

The major blood vessels are extracted on the basis of information of signal intensities of the MRA image. First, a maximum voxel value is selected from a spherical area having a radius of 30 millimeters positioned at the center of the MRA image. Next, initial blood vessel areas included in the sphere area are partitioned using a signal intensity that is 60 percent of the maximum voxel value as a threshold. While observing an increment in the ratio of volumes, an area expansion method is applied to the initial blood vessel areas. In this way, a threshold for the signal intensity is automatically determined, and areas of major blood vessels are acquired from the original MRA image.

A thinning process is performed for the acquired image of major blood vessels. The thinning of the blood vessel image, for example, is performed using "Image J" software. A morphological process is performed as follows.

In order to exclude cavities inside blood vessels, closing processing is performed. Here, the closing processing is a process of repeating execution of contraction processing after expansion processing. The expansion processing is a process of substituting a signal intensity of a target voxel with a maximum signal intensity near the target voxel. The contraction processing is a process of substituting a signal intensity of a target voxel with a minimum signal intensity near the target voxel.

In order to exclude branched blood vessels, contraction processing is performed. A thinned image of major blood vessels is generated by applying a three-dimensional thinning algorithm.

In Step S10, a thinned image SV2 of a clinical case is generated. Coordinate information of brain blood vessels of a patient having cerebral aneurysms and coordinate information of the cerebral aneurysms are associated with the generated thinned image SV2, whereby thinned image information SVI2 is generated. In addition, the thinned image information SVI2 includes size information representing a size of each cerebral aneurysm.

A thinned image SV1 serving as a reference is generated. Coordinate information of brain blood vessels of a patient having cerebral aneurysms and coordinate information of the cerebral aneurysms are associated with the generated thinned image SV1, whereby thinned image information SVI1 is generated.

(Step S20) By performing positioning between the thinned image of the clinical case and the thinned image serving as the reference, the position of the cerebral aneurysm of the clinical case is fitted in the position serving as the reference.

In order to generate an atlas of cerebral aneurysms, it is necessary to perform non-rigid body positioning from a position of a cerebral aneurysm of a clinical case to a position of a thinned image serving as a reference.

First, by using the TPS-RPM method, non-rigid positioning between a set of points of a thinned image of major blood vessels serving as a reference and a set of points of a thinned image of major blood vessels of a clinical case is performed. From this, a deformation vector field defined by points is acquired.

Next, positioning of a position of a cerebral aneurysm of a clinical case in a position of a thinned image of major blood vessels as a reference is performed using a deformation vector at a closest point of the thinned image of the major blood vessels as the reference.

In this way, in Step S20, coordinates of thinned brain blood vessels represented by the thinned image information SVI2 of the clinical case generated in Step S10 are associated with reference coordinates of brain blood vessels represented by the thinned reference image in which the brain blood vessels are thinned that is represented by the thinned image information SVI1 serving as the reference.

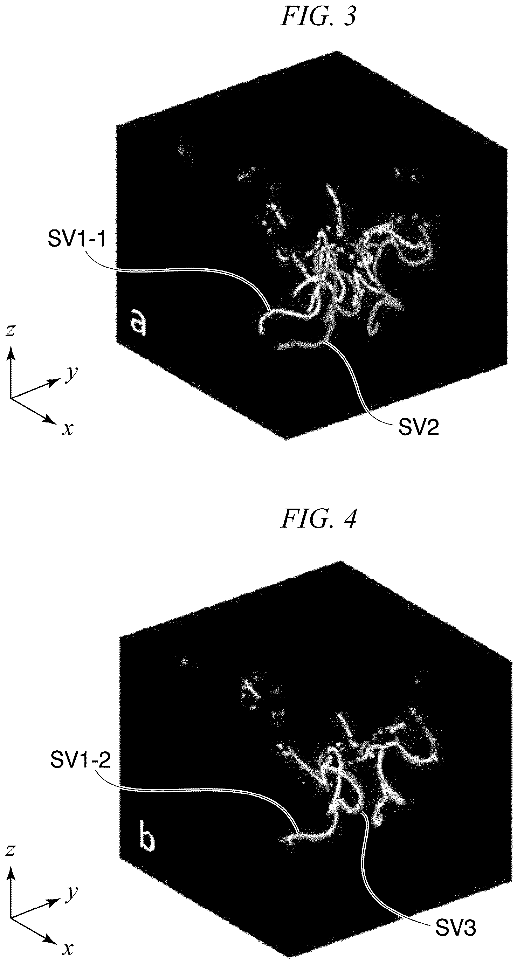

Fig. 3 is a diagram illustrating one example of a thinned image SV1-1 of major blood vessels as a reference and a thinned image SV2 of major blood vessels of a clinical case before positioning is performed. Positions of blood vessels deviate from each other in the thinned image SV1-1 and the thinned image SV2.

Fig. 4 is a diagram illustrating one example of a thinned image SV1-2 of major blood vessels as a reference and a thinned image SV3 of major blood vessels of a clinical case after positioning is performed. Positions of blood vessels in the thinned image SV1-2 and the thinned image SV2 correspond to each other, and deviations therebetween are smaller than those illustrated in Fig. 3.

Referring back to Fig. 2, the description of the process of generating a probabilistic atlas PA will be continued.

(Step S30) A probabilistic atlas PA is generated from the cerebral aneurysms for which the positioning has been completed.

Positions of cerebral aneurysms on the thinned image of major blood vessels as the reference is acquired in accordance with the positioning in Step S20. It is assumed that probabilities of occurrence of cerebral aneurysms surrounding the positions of the cerebral aneurysms follow a normal distribution. The probabilistic atlas PA is generated on the basis of a Gauss distribution represented in Equation (1) around the positions of cerebral aneurysms for which the positioning has been completed.

[Math 1]

$$P_a(v) = \frac{1}{N} \sum_{i=1}^{N} \frac{1}{\left(\sqrt{2\pi\sigma_i^2}\right)^3} \exp\left(-\frac{\|v - c_i\|^2}{2\sigma_i^2}\right) \qquad \cdots \quad (1)$$

Here, a position vector v is a three-dimensional position vector of a position of a voxel, a probability $P_a(v)$ is a probability that a voxel located at a position represented by the position vector v is included in a cerebral aneurysm, a position vector $c_i$ is a position vector of a position of an i-th cerebral aneurysm, and a value $\sigma_i$ is a standard deviation (1/4 of a value of the length of the long diameter of the i-th cerebral aneurysm) of a Gauss distribution.

In this way, in Step S30, a probabilistic atlas PA representing a probability of occurrence at coordinates of a cerebral aneurysm for coordinates of a brain blood vessel represented by the thinned image information SVI1 as the reference is generated on the basis of coordinates of a brain blood vessel associated in coordinate associating step (Step S20) for the thinned image information SVI2 of a plurality of clinical cases and coordinate information.

In addition, in Step S30, a probabilistic atlas PA is generated on the basis of the length of the long diameter of the cerebral aneurysm.

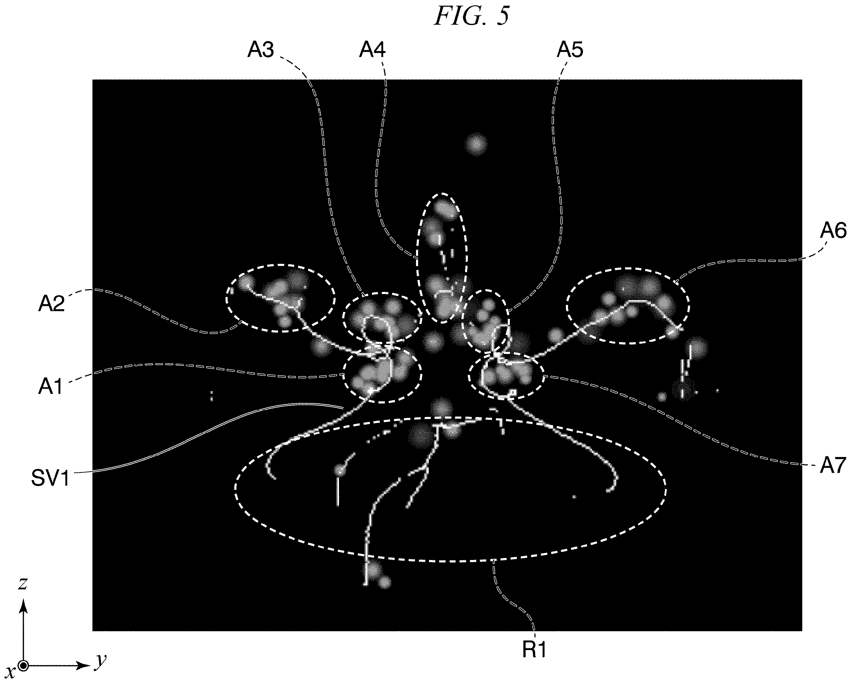

Fig. 5 is a diagram illustrating one example of a probabilistic atlas PA according to this embodiment. The probabilistic atlas PA is generated on the basis of 89 cerebral aneurysms. The probabilistic atlas PA is drawn in a direction in which major blood vessels of a brain are seen from the front side.

A probability distribution of cerebral aneurysms is illustrated on the thinned image SV1 of major blood vessels serving as the reference. For example, at the locations A1, A2, A3, A4, A5, A6, and A7, many cerebral aneurysms occurred in accordance with the many positions of the cerebral aneurysms in clinical cases were distributed here. On the other hand, for example, at the location R1, few cerebral aneurysms occurred in accordance with the few positions of the cerebral aneurysms in clinical cases were distributed here.

(CAD framework)

A CAD framework using Bayesian estimation based on a probabilistic atlas PA will be described.

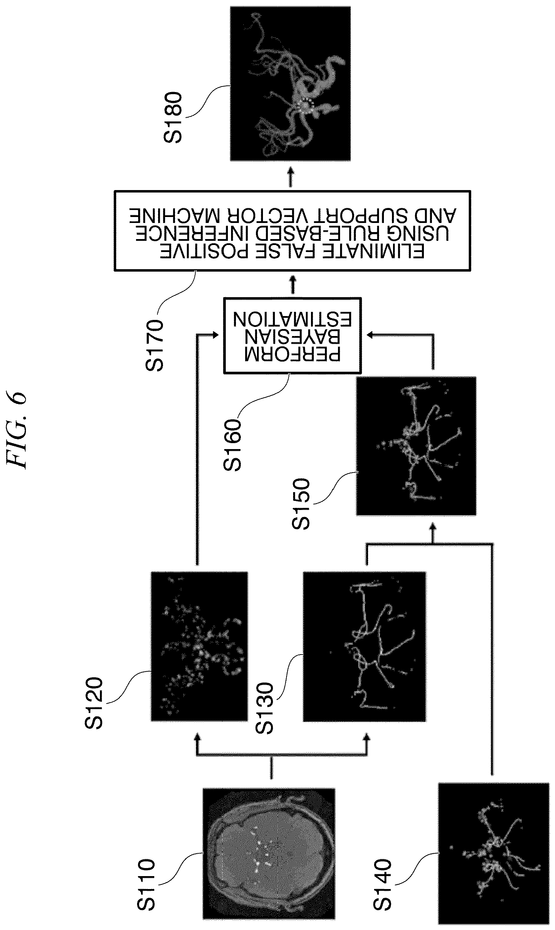

Fig. 6 is a diagram illustrating a CAD framework using Bayesian estimation using a probabilistic atlas PA. Hereinafter, a CAD framework using Bayesian estimation will be referred to as a Bayesian CAD framework.
In Step S110, one MRA image DI of a case for determination is selected from MRA images used for clinical cases and is acquired.
In Step S120, an ECE filter is applied to the MRA image DI acquired in Step S110. In Step S120, the image after application of the ECE filter and a ratio between eigenvalues of Hessian matrices of quadratic equations representing ellipsoids when candidates for cerebral aneurysms are regarded as ellipsoids are acquired. In the image after the application of the ECE filter, shapes of candidates for cerebral aneurysms are selectively enhanced.
In Step S130, determination target thinned image information SVI4 in which coordinate information of brain blood vessels of a patient having a cerebral aneurysm that is a determination target and coordinate information of a cerebral aneurysm are associated with each other, and the brain blood vessels are thinned is generated. Here, a determination target thinned image represented by the determination target thinned image information SVI4 is set as a thinned image SV4.
In Step S140, a probabilistic atlas PA is generated using the map information generating method M represented in Steps S10, S20, and S30.
In Step S150, by performing positioning of the probabilistic atlas PA generated in Step S140 in the position of the thinned image SV4, a probabilistic atlas PA-1 is generated. This positioning is performed similar to the positioning of the thinned image SV2 of major blood vessels of a clinical case in the position of the thinned image SV1 of major blood vessels SV1 as the reference which is represented in Step S20. A location of each cerebral aneurysm represented in a Gauss sphere is aligned in the thinned image SV4 by using a deformation vector field. Here, the deformation vector is acquired by positioning of a blood vessel image using the TPS-RPM method.
A cerebral aneurysm always occurs on the surface of a blood vessel. For this reason, adjustment of local positions is performed for the Gauss sphere of the probabilistic atlas PA. In the thinned image SV4, by moving each Gauss sphere to the surface of a closest blood vessel, a probabilistic atlas PA-1 is acquired.
In Step S160, Bayesian estimation based on the probabilistic atlas PA-1 and a ratio between eigenvalues acquired from the ECE filter is applied to candidates for a cerebral aneurysm. In this way, the number of false positives from the MRA image DI of a case for determination is decreased.
The ECE filter is designed on the basis of eigenvalues of Hessian matrices of a quadratic equation representing an ellipsoid in a voxel represented by a position vector v. A ratio between a maximum value and a minimum value of eigenvalues corresponds to a ratio of a shortest axis to a longest axis in an ellipsoid when the shapes of local signal intensities are modeled as the ellipsoid. This correspondence is represented as in Equation 2.
[Math 2]

$$\frac{(\text{SHORTEST AXIS})}{(\text{LONGEST AXIS})} = \sqrt{\frac{\lambda_1}{\lambda_3}}, 0 \gg \lambda_1 \geq \lambda_2 \geq \lambda_3 \qquad \cdot \cdot \cdot \ (2)$$

Here, eigenvalues $\lambda_1$, $\lambda_2$, and $\lambda_3$ are eigenvalues of the Hessian matrices.
A ratio between the eigenvalues is used as a likelihood in the Bayesian estimation.

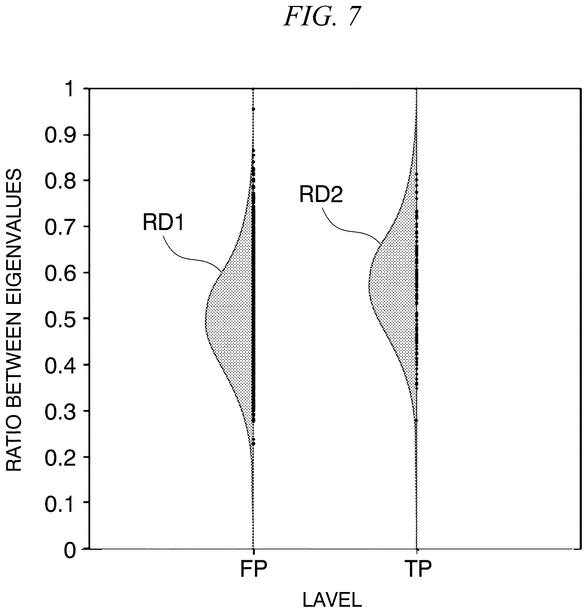

Fig. 7 is diagram illustrating a distribution of ratios of eigenvalues for false positives and cerebral aneurysms. A distribution RD1 of ratios between eigenvalues for cerebral aneurysms is a normal distribution represented by Equation (3).
[Math 3]

$$P_{TP}\big(I(v_{TP})\,|\,v_{TP}\big) = \frac{1}{\sqrt{2\pi\sigma_{TP}^{2}}} \exp\left(-\frac{\big(I(v_{TP})-\mu_{TP}\big)^{2}}{2\sigma_{TP}^{2}}\right) \qquad \cdot \cdot \cdot \ (3)$$

Here, an average value $\mu_{TP}$ is an average value of ratios $I(v_{TP})$ of the eigenvalues, and a dispersion $\sigma_{TP}^2$ is a dispersion of ratios $I(v_{TP})$ between the eigenvalues. A probability $P_{TP}(I(v_{TP}) \mid v_{TP})$ is a probability (likelihood) of the ratio between eigenvalues being the ratio $I(v_{TP})$ in a case in which a position represented by the position vector $v_{TP}$ is included inside a cerebral aneurysm.

A distribution RD2 of ratios between eigenvalues for false positives is a normal distribution represented by Equation (4).

[Math 4]

$$P_{TP}\left(I\!\left(v_{FP}\right)\mid v_{FP}\right)=\frac{1}{\sqrt{2\pi\sigma_{FP}{}^{2}}}\exp\left(-\frac{\left(I\!\left(v_{FP}\right)-\mu_{FP}\right)^{2}}{2\sigma_{FP}{}^{2}}\right) \quad \cdot \cdot \cdot \ (4)$$

Here, the average value $\mu_{FP}$ is an average value of ratios $I(v_{FP})$ of eigenvalues, and the dispersion $\sigma_{FP}^2$ is a dispersion of ratios $I(v_{FP})$ of the eigenvalues. The probability $PFP(I(v_{FP}) \mid v_{FP})$ is a probability (likelihood) of the ratio between eigenvalues being a ratio $I(v_{FP})$ in a case in which a position represented by the position vector $v_{TP}$ is not included inside a cerebral aneurysm.

Referring to Fig. 6, the description of the Bayesian CAD framework will be continued.

In a case in which the ratio $I(v)$ between eigenvalues is acquired, a posteriori probability of a voxel represented by the position vector $v$ being positioned inside a cerebral aneurysm is given as a probability $P(v \mid I(v))$. The probability $P(v \mid I(v))$ is given by Equation (5).

[Math 5]

$$P\left(v\mid I\!\left(v\right)\right)=\frac{P_{TP}\left(I\!\left(v\right)\mid v\ \right)P_a(v)}{P_{TP}\left(I\!\left(v\right)\mid v\ \right)P_a(v)+P_{FP}\left(I\!\left(v\right)\mid v\ \right)P_{FP}(v)}$$

$$=\frac{P_{TP}\left(I\!\left(v\right)\mid v\right)P_a(v)}{P_{TP}\left(I\!\left(v\right)\mid v\right)P_a(v)+P_{FP}\left(I\!\left(v\right)\mid v\right)\left(1-P_a(v)\right)} \quad \cdot \cdot \cdot \ (5)$$

Here, the probability $Pa(v)$ is a probability of occurrence of a cerebral aneurysm given by Equation (1).

The probability distribution of cerebral aneurysms in the probabilistic atlas PA-1 is updated on the basis of the probability of occurrence $P(v \mid I(v))$ of cerebral aneurysms given by Equation (5) acquired in Step S160, whereby a probabilistic atlas PA-2 is generated. By using the probabilistic atlas PA-2, false positives are determined.

In Step S170, false positives are determined on the basis of a rule-based inference and support vector machine (SVM).

For the evaluation, leave-one-out cross validation is used. One is selected from among 89 clinical cases as a case for determination, and the remaining 88 clinical cases are set as clinical cases, and thus the Bayesian CAD framework is executed. This operation is repeated a total of 89 times until all of each of the 89 clinical cases is selected once as a case for determination.

(Result)

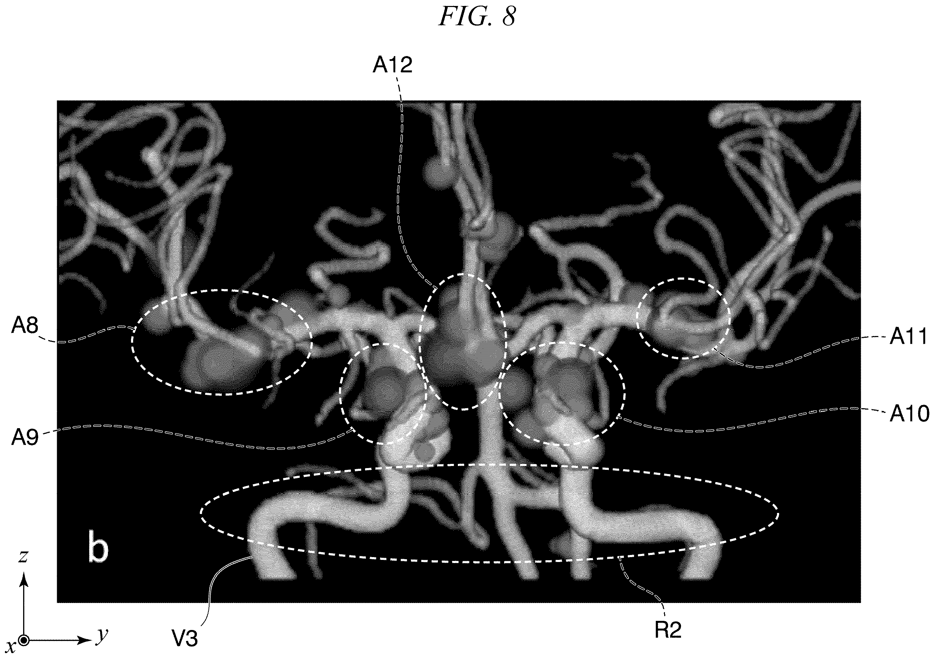

Fig. 8 is a diagram illustrating one example of an image according to a maximum value projecting method that is acquired by viewing the probabilistic atlas PA-2 according to this embodiment from the front side to the rear side.

In results acquired in this example, most of cerebral aneurysms occur to surround a location A12 (a cerebral arterial circle), locations A8 and A11 (MCA), and locations A9 and A10 (ICA bending parts) in a vessel V3. On the other hand, for example, few cerebral aneurysms occur in a location R2.

These results reproduce data representing ratios relating to a size and a location of a cerebral aneurysm in a research of a prevalence rate of unruptured cerebral aneurysm.

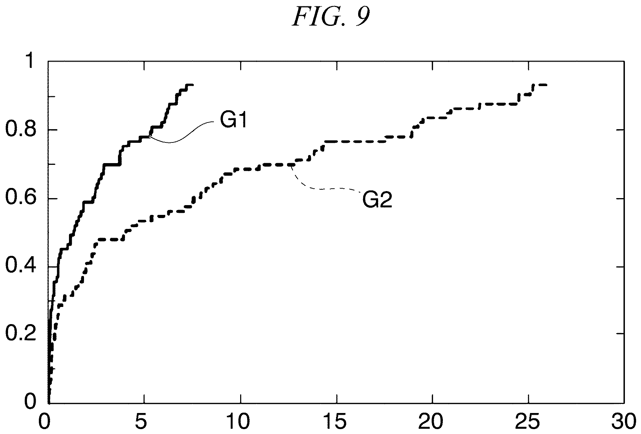

Fig. 9 is a diagram illustrating one example of a FROC curve of a Bayesian CAD framework according to this embodiment. Here, the FROC curve is a curve representing a relation between a false positive and sensitivity.

A free-response receiver operating characteristic (FROC) curve G1 in a case in which Bayesian estimation based on a probabilistic atlas and a ratio between eigenvalues acquired from an ECE filter is used and a FROC curve G2 in a case in which the Bayesian estimation is not used are compared with each other.

According to this embodiment, in a case in which 89 cases are used, when the numbers of false positives in results having the highest sensitivity are compared with each other, it can be confirmed that the initial value of false positives decreases with a sensitivity of 93.1 percent from 25.2 to 7.2 by taking the probabilistic atlas PA into the Bayesian CAD framework. In addition, it can be confirmed that the average number of false positives per case decreases with sensitivity of 80 percent from 19 to 5.4.

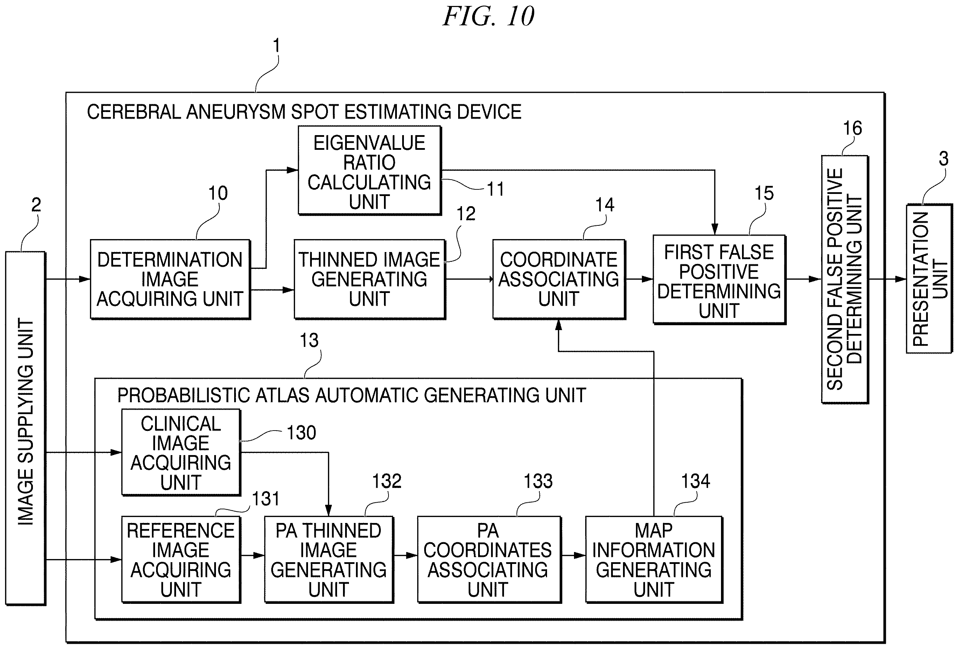

Fig. 10 is a diagram illustrating one example of the functional configuration of a Bayesian CAD framework using a probabilistic atlas according to this embodiment. The Bayesian CAD framework according to this embodiment will be referred to as a frame work F.

The framework F includes a cerebral aneurysm location estimating device 1, an image supplying unit 2, and a presentation unit 3.

The cerebral aneurysm location estimating device 1 includes a determination image acquiring unit 10, an eigenvalue ratio calculating unit 11, a thinned image generating unit 12, a probabilistic atlas automatic generating unit 13, a coordinate associating unit 14, a first false positive determining unit 15, and a second false positive determining unit 16.

The determination image acquiring unit 10 acquires an MRA image DI of brain blood vessels of a patient having a cerebral aneurysm that is a diagnosis target from the image supplying unit 2. The determination image acquiring unit 10 supplies the acquired MRA image DI to the eigenvalue ratio calculating unit 11 and the thinned image generating unit 12.

The eigenvalue ratio calculating unit 11 applies an ECE filter to the MRA image DI acquired from the determination image acquiring unit 10. A ratio between eigenvalues of Hessian matrices used in the ECE filter is calculated for a candidate for each cerebral aneurysm. The eigenvalue ratio calculating unit 11 supplies a ratio between eigenvalues calculated for a candidate for each cerebral aneurysm to the first false positive determining unit 15.

The thinned image generating unit 12 generates a determination target thinned image information SVI4 of major blood vessels from the MRA image DI of a determination target acquired from the determination image acquiring unit 10 and supplies the generated determination target thinned image information SVI4 to the coordinate associating unit 14. Here, the determination target thinned image information SVI4 is information representing a thinned image SV4 including coordinate information of a brain blood vessel that is a determination target.

The probabilistic atlas automatic generating unit 13 automatically generates a probabilistic atlas PA. The probabilistic atlas automatic generating unit 13 includes a clinical image acquiring unit 130, a reference image acquiring unit 131, a PA thinned image generating unit 132, a PA coordinates associating unit 133, and a map information generating unit 134.

The clinical image acquiring unit 130 acquires the MRA image CI of brain blood vessels of a clinical case from the image supplying unit 2. The clinical image acquiring unit 130 supplies the acquired MRA image CI to the PA thinned image generating unit 132.

The reference image acquiring unit 131 acquires an MRA image RI of brain blood vessels serving as a reference from the image supplying unit 2. The clinical image acquiring unit 130 supplies the acquired MRA image RI to the PA thinned image generating unit 132.

The PA thinned image generating unit 132 generates a thinned image information SVI2 from the MRA image CI acquired from the clinical image acquiring unit 130. Here, the thinned image information SVI2 is information representing a thinned image SV2 associated with coordinate information of a brain blood vessel of a patient having a cerebral aneurysm and coordinate information of a cerebral aneurysm. In addition, the thinned image information SVI2 includes size information representing a size of each cerebral aneurysm.

The PA thinned image generating unit 132 generates thinned image information SVI1 from the MRA image RI acquired from the reference image acquiring unit 131. Here, the thinned image information SVI1 is information representing a thinned image SV1 including coordinate information of brain blood vessels of a healthy person serving as a reference.

The PA thinned image generating unit 132 supplies the thinned image information SVI2 and the thinned image information SVI1 that have been generated to the PA coordinates associating unit 133.

The PA coordinates associating unit 133 acquires thinned image information SVI1 and thinned image information SVI2 from the PA thinned image generating unit 132. The PA coordinates associating unit 133 associates coordinates of thinned brain blood vessels represented by the acquired thinned image information SVI2 with reference coordinates of brain blood vessels represented by the acquired thinned image information SVI1.

The PA coordinates associating unit 133 supplies the thinned image information SVI1 and the thinned image information SVI2 in which the coordinates of thinned brain blood vessels are associated with the reference coordinates of the brain blood vessels represented by the thinned image information SVI1 to the map information generating unit 134.

The map information generating unit 134 acquires the thinned image information SVI1 and the thinned image information SVI2 in which the coordinates of the thinned brain blood vessels are associated with reference coordinates of the brain blood vessels represented by the thinned image information SVI1 from the PA coordinates associating unit 133.

The map information generating unit 134 generates a probabilistic atlas PA representing probabilities of occurrence in coordinates of cerebral aneurysm with respect to the reference coordinates of the brain blood vessels represented by the acquires thinned image information SVI1. Here, the map information generating unit 134 generates a probabilistic atlas PA on the basis of the coordinates, the coordinate information, and size information of the cerebral aneurysm represented by the thinned image information SVI2 associated by the PA coordinates associating unit 133.

The map information generating unit 134 supplies the generated probabilistic atlas PA to the coordinate associating unit 14.

The coordinate associating unit 14 acquires the determination target thinned image information SVI4 and the probabilistic atlas PA respectively from the thinned image generating unit 12 and the probabilistic atlas automatic generating unit 13. The coordinate associating unit 14 associates with the coordinates of thinned brain blood vessels represented by the acquired probabilistic atlas PA with the coordinates of brain blood vessels represented by the acquired determination target thinned image information SVI4.

The coordinate associating unit 14 supplies a probabilistic atlas PA-1 associated with the coordinates of the brain blood vessels represented by the determination target thinned image information SVI4 to the first false positive determining unit 15.

The first false positive determining unit 15 acquires the probabilistic atlas PA-1 from the coordinate associating unit 14. The first false positive determining unit 15 performs Bayesian estimation on the basis of the acquired probabilistic atlas PA-1 and the ratio between eigenvalues acquired from the ECE filter. In this way, the first false positive determining unit 15 determines a false positive from among candidates for cerebral aneurysms included in the MRA image DI.

The first false positive determining unit 15 generates a probabilistic atlas PA-2 using Bayesian estimation. The first false positive determining unit 15 determines a false positive from among candidates for cerebral aneurysms included in the MRA image DI using the generated probabilistic atlas PA-2.

The second false positive determining unit 16 determines a false positive from among candidates for cerebral aneurysms included in the MRA image DI on the basis of a rule-based inference and SVM Thereafter, the second false positive determining unit 16 causes the presentation unit 3 to present image information representing a determination result.

The presentation unit 3 acquires image information representing a determination result from the cerebral aneurysm location estimating device 1. The presentation unit 3 causes a display device (not illustrated in the drawing) to display an image of brain blood vessels of a patient having a cerebral aneurysm that is a diagnosis target together with candidates for cerebral aneurysms based on the acquired image information representing the determination result.

(Summary)

In rigid body positioning and positioning based on brain cells, although there is a limit for anatomical differences of locations of blood vessels, it can be recovered through non-rigid body positioning based on a thinned image of blood vessels in accordance with the map information generating method M.

When a probabilistic atlas is generated, positioning of the positions of cerebral aneurysms in the positions of major blood vessels as a reference can be performed without performing manual positioning. For this reason, according to this generation method, chances can be given to substantial users. For example, in a laboratory or a hospital, by using prior information of locations of cerebral aneurysms, an owned database can be used more practically and effectively.

Compared to a manual method of a conventional technology, according to a positioning method using a computer according to this embodiment, the amount of user's operation is decreased, and a difference occurring in accordance with an observer is decreased. The time required for positioning of blood vessels between a thinned image of a clinical case and a thinned image as a reference is one minute 46.2 ± 3.7 seconds in a case in which a general personal computer (a clock number is 3.2 Gigahertz and the number of cores is six) is used. This is a number withstanding daily use.

The CAD framework according to this embodiment can detect 93.1 percent of all the cerebral aneurysms in accordance with 7.2 false positives per case or perform detection with sensitivity of 80 percent in accordance with 5.4 false positives per case.

By employing a magnetic resonance scanner of 3 tesla, a scanning time is decreased by half of that of a magnetic resonance scanner of 1.5 tesla, and accordingly, the scanning can be more comfortable, and a deviation according to movement can be decreased.

The map information generating method M relating to the embodiment described above includes: a thinned patient image generating step (Step S10) of generating thinned patient image information (the thinned image information SVI2) in which coordinate information of brain blood vessels of a patient having a cerebral aneurysm and coordinate information of cerebral aneurysms are associated with each other, and the brain blood vessels are thinned; a coordinate associating step (Step S20) of associating coordinates of thinned blood vessels represented by the thinned patient image information (the thinned image information SVI2) generated in the thinned patient image generating step (Step S10) with reference coordinates of brain blood vessels represented by a thinned reference image (the thinned images SV1-1 and SV1-2) in which the brain blood vessels are thinned; and a map information generating step (Step S30) of generating probabilistic map information (the probabilistic atlas PA) representing probabilities of occurrence in the coordinates of cerebral aneurysms with respect to the reference coordinates (the coordinates of brain blood vessels represented by the thinned image information SVI1 as a reference) on the basis of the coordinates of the brain blood vessels associated in the coordinate associating step (Step S20) for the thinned patient image information (the thinned image information SVI2) of a plurality of patients having cerebral aneurysms and the coordinate information. Therefore, according to the map information generating method M, a map representing probabilities of occurrence of cerebral aneurysms can be automatically generated.

According to the map information generating method M relating to the embodiment described above, the probabilistic map information (probabilistic atlas PA) is generated on the basis of sizes of the cerebral aneurysms (lengths of long diameters of the cerebral aneurysms). Therefore, according to the map information generating method M, information of the sizes of the cerebral aneurysms can be reflected on the map representing probabilities of occurrence of cerebral aneurysms.

A determination method according to the embodiment described above includes: a determination target thinned patient image generating step (Step S130) of generating determination target thinned patient image information (the determination target thinned image information SVI4) in which coordinate information of brain blood vessels of a patient having a cerebral aneurysm that is a determination target and coordinate information of cerebral aneurysms are associated with each other, and the brain blood vessels are thinned; a probabilistic map information (probabilistic atlas PA) generating step (Step S140) of generating the probabilistic map information using the map information generating method M; a second coordinate associating step (Step S150) of associating coordinates of thinned brain blood vessels represented by the probabilistic map information (the probabilistic atlas PA) generated in the probabilistic map information generating step (Step S140) with reference coordinates of the brain blood vessels (coordinates of brain blood vessels represented by the determination target thinned image information SVI4) represented by the determination target thinned patient image information (the determination target thinned image information SVI4); a second map information generating step (Step S150) of generating second probabilistic map information (the probabilistic atlas PA-1) on the basis of the coordinates of the brain blood vessels associated in the second coordinate associating step (Step S150) and the coordinate information; and a false positive determining step of determining a false positive included in the second probabilistic map information (the probabilistic atlas PA-1) using Bayesian estimation. Therefore, according to the determination method relating to the embodiment described above, the number of false positives can be decreased in the determination result.

According to the determination method relating to the embodiment described above, a ratio between eigenvalues of Hessian matrices of quadratic equations representing ellipsoids when cerebral aneurysms are regarded as the ellipsoids is used. Therefore, according to the determination method relating to the embodiment described above, the number of false positives in the determination result can be decreased on the basis of the shapes of cerebral aneurysms.

While the preferred embodiment of the invention has been described with reference to the drawings, a specific configuration is not limited to this embodiment, and an appropriate change can be made therein in a range not departing from the concept of the present invention. The configurations described in the embodiments described above may be combined.

In addition, the present invention can be applied to the generation of a probabilistic map of blood vessels, tracheas, and lung tissues of parts in which a lung cancer frequently occurs. In such a case, for example, a probabilistic map is generated with major blood vessels of the brain blood vessels described in the embodiment described above being in correspondence with the blood vessels and tracheas. In this way, the present invention can be applied to organs having a common structure between individuals by performing positioning of positions of infected parts in an organ serving as a reference. For example, the present invention may be applied to organs such as skeletons, major nerve systems, major lymph vessels, alimentary canals, and the like.

In addition, each unit included in each device according to the embodiment described above may be realized by dedicated hardware and may be realized by a memory and a microprocessor.

Furthermore, each unit included in each device may be configured by a memory and a central processing unit (CPU), and the function thereof may be realized by loading a program used for realizing the function of each unit included in each device into a memory and executing the program.

In addition, a program used for realizing the function of each unit included in each device may be recorded on a computer-readable recording medium, and a process using each unit included in a control unit may be performed by causing a computer system to read the program recorded on this recording medium and execute the program. A "computer system" described here includes an OS and hardware such as peripherals and the like.

In addition, the "computer system" also includes a home page providing environment (or a display environment) in a case in which a WWW system is used.

A "computer-readable recording medium" represents a storage device including a portable medium such as a flexible disk, a magneto-optical disc, a ROM, or a CD-ROM, a hard disk built in a computer system, and the like. Furthermore, a "computer-readable recording medium" also includes a medium dynamically storing the program for a short time such as a communication line in a case in which the program is transmitted through a network such as the Internet or a communication circuit line such as a telephone circuit line and a medium storing the program for a fixed time such as a volatile memory inside a computer system serving as a server or a client in such a case. In addition, the program described above may be used for realizing a part of the functions described above and, furthermore, may be a program realizing the functions described above by being combined with a program recorded in the computer system in advance.

[Reference Signs List]

1 Cerebral aneurysm location estimating device
2 Image supplying unit
3 Presentation unit
10 Determination image acquiring unit
11 Eigenvalue ratio calculating unit
12 Thinned image generating unit
13 Probabilistic atlas automatic generating unit
14 Coordinate associating unit
15 First false positive determining unit
16 Second false positive determining unit
130 Clinical image acquiring unit
131 Reference image acquiring unit
132 PA thinned image generating unit
133 PA coordinates associating unit
134 Map information generating unit
VI Blood vessel
FP1 Entrance part
FP2, FP3 Terminal end
A1, A2, A3, A4, A5, A6, A7, R1 Spot
SV1-1, SV1-2, SV2, SV3 Thinned image

**Claims**

1. A method for generating map information comprising:

   a thinned patient image generating step of generating thinned patient image information in which coordinate information of brain blood vessels of a patient having a cerebral aneurysm and coordinate information of cerebral aneurysms are associated with each other, and the brain blood vessels are thinned;
   a coordinate associating step of associating coordinates of thinned blood vessels represented by the thinned patient image information generated in the thinned patient image generating step with reference coordinates of brain blood vessels represented by a thinned reference image in which the brain blood vessels are thinned; and
   a map information generating step of generating probabilistic map information representing probabilities of occurrence in the coordinates of cerebral aneurysms with respect to the reference coordinates on the basis of the coordinates of the brain blood vessels associated in the coordinate associating step for the thinned patient image information of a plurality of patients having cerebral aneurysms and the coordinate information.

2. The method for generating map information according to claim 1, wherein the probabilistic map information is generated on the basis of sizes of the cerebral aneurysms in the map information generating step.

3. A determination method comprising:

   a determination target thinned patient image generating step of generating determination target thinned patient image information in which coordinate information of brain blood vessels of a patient having a cerebral aneurysm that is a determination target and coordinate information of cerebral aneurysms are associated with each other, and the brain blood vessels are thinned;
   a probabilistic map information generating step of generating the probabilistic map information using the method for generating map information according to claim 1 or 2;
a second coordinate associating step of associating coordinates of thinned brain blood vessels represented by the probabilistic map information generated in the probabilistic map information generating step with reference coordinates of the brain blood vessels represented by the determination target thinned patient image information;
a second map information generating step of generating second probabilistic map information on the basis of the coordinates of the brain blood vessels associated in the second coordinate associating step and the coordinate information; and
a false positive determining step of determining a false positive included in the second probabilistic map information using Bayesian estimation.

4. The determination method according to claim 3, wherein a ratio between eigenvalues of Hessian matrices of quadratic equations representing ellipsoids when cerebral aneurysms are regarded as ellipsoids is used in the false positive determining step.

5. A program causing a computer to execute:

a thinned patient image generating step of generating thinned patient image information in which coordinate information of brain blood vessels of a patient having a cerebral aneurysm and coordinate information of cerebral aneurysms are associated with each other, and the brain blood vessels are thinned;
a coordinate associating step of associating coordinates of thinned blood vessels represented by the thinned patient image information generated in the thinned patient image generating step with reference coordinates of brain blood vessels represented by a thinned reference image in which the brain blood vessels are thinned; and
a map information generating step of generating probabilistic map information representing probabilities of occurrence in the coordinates of cerebral aneurysms with respect to the reference coordinates on the basis of the coordinates of the brain blood vessels associated in the coordinate associating step for the thinned patient image information of a plurality of patients having cerebral aneurysms and the coordinate information.

FIG. 1

EP 3 632 306 A1

*FIG. 2*

```
        ┌─────────────┐
        │    START    │
        └─────────────┘
               │
               ▼              S10
┌──────────────────────────────────┐
│  EXTRACT MAJOR BRAIN BLOOD VESSEL │
│   AND THIN MAJOR BLOOD VESSEL     │
└──────────────────────────────────┘
               │
               ▼              S20
┌──────────────────────────────────┐
│ PERFORM POSITIONING OF THINNED    │
│   IMAGE OF MAJOR BLOOD VESSELS    │
│       OF CLINICAL CASE            │
│   IN POSITIONS OF THINNED IMAGE   │
│           OF MAJOR BLOOD          │
│   VESSELS SERVING AS REFERENCE    │
└──────────────────────────────────┘
               │
               ▼              S30
┌──────────────────────────────────┐
│   GENERATE CEREBRAL ANEURYSM      │
│      PROBABILISTIC ATLAS BY       │
│  POSITIONING CEREBRAL ANEURYSM    │
└──────────────────────────────────┘
               │
               ▼
        ┌─────────────┐
        │     END     │
        └─────────────┘
```

## *FIG. 3*

SV1-1

SV2

z

y

x

a

## *FIG. 4*

SV1-2

SV3

z

y

x

b

*FIG. 5*

EP 3 632 306 A1

*FIG. 6*

S110

S120

S130

S140

S150

PERFORM
BAYESIAN
ESTIMATION

S160

ELIMINATE FALSE POSITIVE
USING RULE-BASED INFERENCE
AND SUPPORT VECTOR MACHINE

S170

S180

*FIG. 7*

FIG. 8

FIG. 9

FIG. 10

# EP 3 632 306 A1

| | | | |
|---|---|---|---|

**INTERNATIONAL SEARCH REPORT**

International application No.

PCT/JP2018/018115

| A. CLASSIFICATION OF SUBJECT MATTER |
|---|
| Int. Cl. A61B5/055(2006.01)i, A61B5/00(2006.01)i |
| According to International Patent Classification (IPC) or to both national classification and IPC |

| B. FIELDS SEARCHED |
|---|
| Minimum documentation searched (classification system followed by classification symbols) |
| Int. Cl. A61B5/055, A61B5/00, A61B6/00-6/14, G06T7/00, G06Q50/22 |
| Documentation searched other than minimum documentation to the extent that such documents are included in the fields searched |
| Published examined utility model applications of Japan 1922-1996<br>Published unexamined utility model applications of Japan 1971-2018<br>Registered utility model specifications of Japan 1996-2018<br>Published registered utility model applications of Japan 1994-2018 |
| Electronic data base consulted during the international search (name of data base and, where practicable, search terms used) |

| C. DOCUMENTS CONSIDERED TO BE RELEVANT | | |
|---|---|---|
| Category* | Citation of document, with indication, where appropriate, of the relevant passages | Relevant to claim No. |
| A | US 2015/0317442 A1 (SIEMENS AKTIENGESELLSCHAFT) 05 November 2015, fig. 1, paragraphs [0036], [0037] (Family: none) | 1-5 |
| A | WO 2017/047819 A1 (EBM CORP.) 23 March 2017, entire text, all drawings (Family: none) | 1-5 |
| A | WO 2013/031741 A1 (EBM CORP.) 07 March 2013, entire text, all drawings & US 2014/0316758 A1, entire text, all drawings & EP 2749223 A1 & CA 2850189 A1 & CN 103917165 A & KR 10-2014-0082663 A | 1-5 |
| A | JP 2008-73338 A (GIFU UNIVERSITY) 03 April 2008, entire text, all drawings (Family: none) | 1-5 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 23.07.2018 | 31.07.2018 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2018/018115

C (Continuation).    DOCUMENTS CONSIDERED TO BE RELEVANT

| Category* | Citation of document, with indication, where appropriate, of the relevant passages | Relevant to claim No. |
|---|---|---|
| A | JIN, Ze, An ellipsoid convex enhancement filter for detection of asymptomatic intracranial aneurysm candidates in CAD frameworks, Med. Phys., February 2016, vol. 43, no. 2, 951-960 | 1-5 |

Form PCT/ISA/210 (continuation of second sheet) (January 2015)

**EP 3 632 306 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017106643 A

- JP 2009240543 A